# EUROPÄISCHE PATENTSCHRIFT

(11) **EP 3 796 135 B1**
(45) Veröffentlichungstag und Bekanntmachung des Hinweises auf die Patenterteilung: **24.01.2024**
(21) Anmeldenummer: 20186161.4
(22) Anmeldetag: 16.07.2020
(51) Int. Cl.: G06F 3/01, G06Q 10/00, G06Q 50/02, H04N 21/414, H04W 4/02, G06T 19/00

(54) **VERFAHREN ZUR UNTERSTÜTZUNG EINES BENUTZERS BEI EINER LANDWIRTSCHAFTLICHEN TÄTIGKEIT**
METHOD FOR ASSISTING A USER INVOLVED IN AN AGRICULTURAL ACTIVITY
PROCÉDÉ PERMETTANT D'AIDER UN UTILISATEUR À UNE ACTIVITÉ AGRICOLE

(30) Priorität: 20.09.2019 DE 102019125348
(43) Veröffentlichungstag der Anmeldung: 24.03.2021
(73) Patentinhaber: 365FarmNet Group KGaA mbh & Co KG, 33428 Harsewinkel (DE)
(72) Erfinder: Weiß, Philipp, 10318 Berlin (DE)
(74) Vertreter: CLAAS Gruppe

(56) Entgegenhaltungen:
- WO-A1-2015/161307
- DE-A1-102014 103 195
- US-A1- 2016 140 728
- US-A1- 2017 097 413
- US-A1- 2017 124 776
- US-A1- 2017 140 457
- US-A1- 2017 322 715
- US-A1- 2018 088 663
- US-A1- 2018 088 677
- US-A1- 2018 131 907
- US-A1- 2018 150 070
- US-A1- 2018 189 568
- US-A1- 2019 147 655

## Beschreibung

Die Erfindung betrifft ein Verfahren zur Unterstützung eines Benutzers bei einer landwirtschaftlichen Tätigkeit gemäß dem Oberbegriff von Anspruch 1, ein mobiles Gerät ausgestaltet zur Verwendung in dem vorschlagsgemäßen Verfahren gemäß Anspruch 14 sowie eine Serveranwendung ausgestaltet zur Verwendung in dem vorschlagsgemäßen Verfahren gemäß Anspruch 15.

Diverse landwirtschaftliche Tätigkeiten werden inzwischen datengestützt durchgeführt. Eine Möglichkeit, Landwirte und andere Benutzer bei derartigen Tätigkeiten zu unterstützen, sind augmented reality-Routinen. Diese werden üblicherweise mit Hilfe von mobilen Geräten und häufig unterstützt von Serveranwendungen durchgeführt. Aufgrund der hohen Komplexität sowohl der Daten als auch der Tätigkeiten, ist es von Vorteil, wenn die augmented reality-Routine den Benutzer möglichst intuitiv und einfach unterstützt.

Aus dem Stand der Technik (DE 10 2014 103 195 A1, DE 10 2016 215 199 A1, EP 3 166 054 A1) ist es bekannt, mit Hilfe von mobilen Geräten Zusatzinformationen nach Art einer augmented reality-Routine auf einem Display des mobilen Geräts einem von einer Kamera erzeugten Abbild der Realität zu überlagern. Es ist aus dem Stand der Technik auch bekannt, derartige augmented reality-Routinen in einem landwirtschaftlichen Zusammenhang durchzuführen. Es ist jedoch eine Vielzahl landwirtschaftlicher Szenarien denkbar, in denen der Benutzer mittels augmented reality-Routinen unterstützt werden kann. Diese landwirtschaftlichen Szenarien weisen zum Teil nur sehr wenige Gemeinsamkeiten auf. Entsprechend sind auch die anzuzeigenden Zusatzinformationen sowie die notwendigen Berechnungen des jeweiligen Szenarios sehr unterschiedlich. Eine augmented reality-Routine, die jegliches landwirtschaftliche Szenario abdeckt, ist daher nicht ohne weiteres möglich. Bisher beziehen sich augmented reality-Routinen daher auf einzelne landwirtschaftliche Szenarien, sind aber nicht dazu gedacht, den Benutzer vollumfänglich während möglichst vieler landwirtschaftlicher Szenarien zu unterstützen.

Folgende Dokumente könnten relevanten Stand der Technik beinhalten: US 2018/131907 A1, US 2018/088677 A1, US 2019/147655 A1, US 2017/097413 A1, WO 2015/161307 A1, US 2018/088663 A1, US 2016/140728 A1, US 2018/189568 A1 und US 2017/322715 A1.

Der Erfindung liegt das Problem zugrunde, das bekannte Verfahren derart auszugestalten und weiterzubilden, dass sie bei möglichst geringer Komplexität möglichst flexibel in verschiedenen landwirtschaftlichen Szenarien einsetzbar sind.

Das obige Problem wird bei einem Verfahren gemäß dem Oberbegriff von Anspruch 1 durch die Merkmale des kennzeichnenden Teils von Anspruch 1 gelöst.

Wesentlich ist die grundsätzliche Überlegung, dass eine zumindest ein mobiles Gerät und eine mit dem mobilen Gerät kommunizierende Serveranwendung aufweisende Steueranordnung so ausgestaltet ist, dass sie automatisch ermittelt, in welchem landwirtschaftlichen Szenario sich das mobile Gerät befindet. Dafür umfasst die Serveranwendung mehrere Anwendungsbausteine, denen jeweils ein landwirtschaftliches Szenario zugeordnet ist. Beispiele, die im Weiteren noch näher erläutert werden, sind ein landwirtschaftliches Szenario "Feldinspektion", ein landwirtschaftliches Szenario "Tierbestandsinspektion" und ein landwirtschaftliches Szenario "Feldbearbeitung". Durch das Zusammenspiel zwischen dem mobilen Gerät und der Serveranwendung kann der Benutzer dann abhängig von dem jeweiligen landwirtschaftlichen Szenario spezifisch unterstützt werden.

Im Einzelnen wird vorgeschlagen, dass die Serveranwendung mehrere Anwendungsbausteine, denen jeweils ein vordefiniertes landwirtschaftliches Szenario zugeordnet ist, umfasst und in der Datenbank zu jedem landwirtschaftlichen Szenario eine Menge von Zusatzinformationen gespeichert ist, dass von der Steueranordnung automatisch ermittelt wird, in welchen der vordefinierten landwirtschaftlichen Szenarien sich das mobile Gerät befindet, dass von der Steueranordnung abhängig von dem ermittelten landwirtschaftlichen Szenario die Menge in der Datenbank gespeicherter Zusatzinformationen ermittelt wird und dass abhängig von mindestens einem in dem Abbild der Realität abgebildeten Objekt eine Teilmenge der in der Datenbank zu dem ermittelten landwirtschaftlichen Szenario gespeicherten Menge von Zusatzinformationen auf dem Display angezeigt wird.

Das vorschlagsgemäße Verfahren weist somit den Vorteil auf, dass der Benutzer nicht mit einer Menge für ihn zu einem bestimmten Zeitpunkt unnötiger Zusatzinformationen überflutet wird und deshalb die Steueranordnung gleichzeitig nicht eine große Menge an Berechnungen durchführen muss, deren Ergebnis für den Benutzer zu dem jeweiligen Zeitpunkt irrelevant wäre. Erst dadurch wird es möglich, den Benutzer in einer Vielzahl landwirtschaftlicher Szenarien jeweils bedarfsgerecht zu unterstützen, ohne dabei die Komplexität für den Benutzer zu erhöhen.

Bei der bevorzugten Ausgestaltung gemäß Anspruch 2 sind den Anwendungsbausteinen jeweils Identifikationsbedingungen zugeordnet, die mit dem mobilen Gerät zugeordneten Identifikationsinformationen abgeglichen werden, um zu ermitteln, in welchem landwirtschaftlichen Szenario sich das mobile Gerät befindet. Schon eine kleine Menge an Identifikationsinformationen kann ausreichen, um viele landwirtschaftliche Szenarien auszuschließen oder direkt das aktuelle landwirtschaftliche Szenario zu ermitteln. So wird unter Einsatz von nur geringer Rechenleistung schnell automatisch ermittelt, in welchem landwirtschaftlichen Szenario sich das mobile Gerät befindet. Dadurch wird es gleichzeitig möglich, den Benutzer in einer Vielzahl potentieller landwirtschaftlicher Szenarien zu unterstützen, ohne dass die Auswahl des tatsächlich aktuellen landwirtschaftlichen Szenarios unmöglich oder sehr langwierig wird.

Bei einer weiter bevorzugten Ausgestaltung gemäß Anspruch 3 umfassen die Identifikationsbedingungen das Vorhandensein von Identifikations-Objekten der realen Welt. Beispielsweise kann durch das Vorhandensein spezifischer Tiere als Identifikations-Objekte schnell auf ein tierspezifisches landwirtschaftliches Szenario geschlossen werden, während das Vorhandensein eines Mähdreschers und eines erntereifen landwirtschaftlichen Feldes als Identifikations-Objekte auf ein erntespezifisches landwirtschaftliches Szenario schließen lassen.

Um beispielsweise das Vorhandensein einer landwirtschaftlichen Arbeitsmaschine unkompliziert ermitteln zu können, wird bei der Ausgestaltung gemäß Anspruch 4 vorgeschlagen, dass das mobile Gerät über eine Funkverbindung mit zumindest einem Objekt der realen Welt verbunden ist. Schon das Vorhandensein der Funkverbindung, aber auch darüber übermittelte Identifikationsinformationen, können zur Ermittlung des landwirtschaftlichen Szenarios herangezogen werden.

Bei herkömmlichen augmented reality-Routinen wird von der Steueranordnung häufig nur über Bilderkennung ermittelt, welche Objekte der realen Welt potentiell in der Umgebung des mobilen Geräts vorhanden sein könnten. Vorliegend wurde nun jedoch erkannt, dass die Menge potentieller Funkverbindungen in landwirtschaftlichen Szenarien, verglichen mit der Menge potentieller durch Bilderkennung zu erkennender Objekte, sehr gering ist. Da die Erkennung einer Funkverbindung sowie das Übertragen von Daten über diese Funkverbindung außerdem weniger Rechenleistung benötigt als eine Bilderkennung, stellt das Hinzunehmen der Funkverbindung bei der Ermittlung des landwirtschaftlichen Szenarios eine sehr gute Möglichkeit dar, die Menge potentieller landwirtschaftlicher Szenarien schnell zu begrenzen.

Die Ansprüche 5, 6 und 7 betreffen die bevorzugten landwirtschaftlichen Szenarien "Feldinspektion", Tierbestandsinspektion" und "Feldbearbeitung". Diese landwirtschaftlichen Szenarien profitieren in besonderer Art und Weise von den Möglichkeiten der Unterstützung mittels augmented reality-Routinen, während die in ihnen vom Benutzer benötigten Zusatzinformationen gleichzeitig deutlich unterschiedlich sind. Durch die automatische Ermittlung, in welchem dieser vordefinierten landwirtschaftlichen Szenarien sich das mobile Gerät befindet, kann der Benutzer so in jedem der landwirtschaftlichen Szenarien szenariospezifisch unterstützt werden.

Anspruch 8 gibt bevorzugte Ausführungsformen des mobilen Geräts an.

Gemäß Anspruch 9 kann vorgesehen sein, dass der Benutzer durch Eingaben an dem mobilen Gerät die angezeigten Zusatzinformationen modifizieren kann. Dabei kann es sich um einfache Informationen, wie zum Beispiel die vorgenommene Fütterung eines Tieres, handeln, die so in der Datenbank abgespeichert werden. Genauso vorteilhaft kann jedoch vorgesehen sein, dass der Benutzer komplexe Planungen direkt vor Ort eingeben kann. Dadurch kann der Benutzer die Vorteile der augmented reality vollständig ausnutzen.

Anspruch 10 betrifft Identifikationsinformationen, die insbesondere dem landwirtschaftlichen Szenario "Feldinspektion" zugeordnet sein können. Da allgemein viele der landwirtschaftlichen Szenarien nur an bestimmten Orten kommen können, kommt insbesondere Positionsinformationen des mobilen Geräts als Identifikationsinformationen eine große Bedeutung zu.

Anspruch 11 betrifft Identifikationsinformationen, die insbesondere für das landwirtschaftliche Szenario "Tierbestandsinspektion" relevant sein können. Bemerkenswert ist hier insbesondere die Tatsache, dass Tiere im landwirtschaftlichen Betrieb häufig mit RFID-Tags gekennzeichnet sind, deren Vorhandensein allein schon viele Informationen liefert und die zusätzlich Rückschlüsse auf das spezifische Tier erlauben.

Anspruch 12 betrifft die Möglichkeit, dass das landwirtschaftliche Szenario "Feldbearbeitung" und das landwirtschaftliche Szenario "Feldinspektion" miteinander verbunden sein können.

Anspruch 13 betrifft bevorzugte Ausführungsformen der Anwendungsbausteine.

Nach einer weiteren Lehre gemäß Anspruch 14, der eigenständige Bedeutung zukommt, wird ein mobiles Gerät ausgestaltet zur Verwendung in dem vorschlagsgemäßen Verfahren beansprucht. Auf alle Ausführungen zu dem vorschlagsgemäßen Verfahren darf verwiesen werden.

Nach einer weiteren Lehre gemäß Anspruch 15, der ebenfalls eigenständige Bedeutung zukommt, wird eine Serveranwendung ausgestaltet zur Verwendung in einem vorschlagsgemäßen Verfahren beansprucht. Auf alle Ausführungen zu dem vorschlagsgemäßen Verfahren darf verwiesen werden.

Im Folgenden wird die Erfindung anhand einer lediglich ein Ausführungsbeispiel darstellenden Zeichnung näher erläutert. In der Zeichnung zeigt
- Fig. 1: das vorschlagsgemäße Verfahren im Einsatz an einem Feld,
- Fig. 2: das mobile Gerät bei der Nutzung im vorschlagsgemäßen Verfahren an einem Feld,
- Fig. 3: das mobile Gerät bei der Nutzung im vorschlagsgemäßen Verfahren in einem Stall und
- Fig. 4: das mobile Gerät bei der Nutzung im vorschlagsgemäßen Verfahren während der Bearbeitung eines Felds.

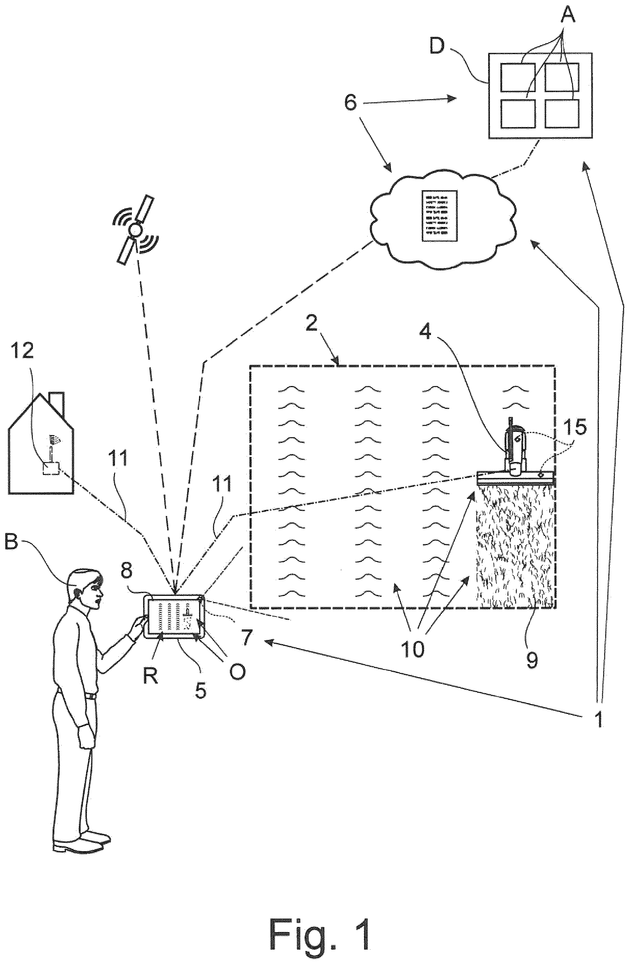

Fig. 1 zeigt schematisch einen möglichen Einsatz des vorschlagsgemäßen Verfahrens zur Unterstützung eines Benutzers B bei einer landwirtschaftlichen Tätigkeit. Der Benutzer B wird hier mittels einer Steueranordnung 1 unterstützt. Der Begriff "landwirtschaftliche Tätigkeit" ist dabei weit zu verstehen und umfasst jegliche Tätigkeit, die in Zusammenhang mit der zielgerichteten Herstellung pflanzlicher und/oder tierischer Erzeugnisse steht. Einige Beispiele für landwirtschaftliche Tätigkeiten sind ein Inspizieren eines Feldes 2 oder eines Tierbestands 3, aber auch ein Abernten des Feldes 2, ein Pflügen des Feldes 2 oder eine Wartung einer landwirtschaftlichen Arbeitsmaschine 4.

Die Steueranordnung 1 weist zumindest ein mobiles Gerät 5 und eine mit dem mobilen Gerät 5 kommunizierende Serveranwendung 6 mit einer Datenbank D auf. Der Begriff "Serveranwendung" ist dabei nicht im engen Sinne als Computerprogramm zu verstehen, sondern umfasst auch einen oder mehrere Server, auf denen ein entsprechendes Computerprogramm ausgeführt wird und auf denen die Datenbank D abgespeichert sein kann.

Das mobile Gerät 5, das im in den Figuren dargestellten Ausführungsbeispiel ein Tablet ist, weist eine Kamera 7 und ein Display 8 auf. Weitere bevorzugte Ausführungsformen des mobilen Geräts 5 werden im Folgenden noch genannt.

Von der Steueranordnung 1 wird eine augmented reality-Routine ausgeführt. Dafür können grundsätzlich bekannte Hilfsmittel wie beispielsweise ARCore genutzt werden. In der augmented reality-Routine werden auf dem Display 8 des mobilen Geräts 5 ein von der Kamera 7 erzeugtes Abbild der Realität R und mindestens eine in der Datenbank D gespeicherte Zusatzinformation Z, die einem landwirtschaftlichen Szenario zugeordnet ist, visuell überlagert angezeigt. Bei dem Abbild der Realität R handelt es sich üblicherweise um normale Videodaten. Es kann sich jedoch auch um ein Foto oder bearbeitete Videodaten handeln. Hier und vorzugsweise handelt es sich um live-Videodaten.

Die jeweilige Zusatzinformation Z ist mindestens einem in dem Abbild der Realität R abgebildeten Objekt O der realen Welt zugeordnet. Das in dem Abbild der Realität R abgebildete Objekt O der realen Welt kann beispielsweise ein abgebildetes Tier, eine abgebildete Pflanze, oder ein abgebildeter Abschnitt eines Feldes 2 sein.

Es ist nun vorschlagsgemäß so, dass die Serveranwendung 1 mehrere Anwendungsbausteine A, denen jeweils ein vordefiniertes landwirtschaftliches Szenario zugeordnet ist, umfasst. In der Datenbank D ist zu jedem landwirtschaftlichen Szenario eine Menge von Zusatzinformationen Z gespeichert.

Von der Steueranordnung 1 wird automatisch ermittelt, in welchem der vordefinierten landwirtschaftlichen Szenarien sich das mobile Gerät 5 befindet. Diese automatische Ermittlung kann es dem Benutzer B ersparen, bei jedem Wechsel eines landwirtschaftlichen Szenarios selbst eine oder mehrere der Anwendungsbausteine A auswählen zu müssen, möglicherweise selbst in Unkenntnis darüber, welcher der Anwendungsbausteine A ihm die benötigten Zusatzinformationen Z verschafft.

Abhängig von dem ermittelten landwirtschaftlichen Szenario wird von der Steueranordnung 1 die Menge in der Datenbank D gespeicherter Zusatzinformationen Z ermittelt. Dabei kann vorgesehen sein, dass alle in der Datenbank D gespeicherten Zusatzinformationen Z einem Anwendungsbaustein A zugeordnet sind, wodurch die Steueranordnung 1 mit wenig Aufwand die in dem ermittelten landwirtschaftlichen Szenario relevanten Zusatzinformationen Z ermitteln kann.

Insbesondere um zu vermeiden, dass der Benutzer B eine zu große Menge an Zusatzinformationen Z angezeigt bekommt, die ihm zum jeweiligen Zeitpunkt nicht nützlich sind, wird abhängig von mindestens einem in dem Abbild der Realität R abgebildeten Objekt O eine Teilmenge der in der Datenbank D zu dem ermittelten landwirtschaftlichen Szenario gespeicherten Menge von Zusatzinformationen Z auf dem Display 8 angezeigt. Zur Ermittlung der Teilmenge kann beispielsweise eine Bilderkennungs-Routine vorgesehen sein, die zumindest einige der in dem Abbild der Realität R abgebildeten Objekte O identifiziert. Alternativ oder zusätzlich nutzbare Möglichkeiten, die abgebildeten Objekte O zu identifizieren, werden im Folgenden noch erläutert.

Das vorschlagsgemäße Verfahren zeichnet sich somit durch seine Zweistufigkeit aus. Zuerst wird ermittelt, in welchem der vordefinierten landwirtschaftlichen Szenarien sich das mobile Gerät 5 befindet, und dann wird der Benutzer B abhängig von dem landwirtschaftlichen Szenario unterstützt. Dabei ist jedoch nicht ausgeschlossen, dass das Verfahren vollständig oder teilweise mehrfach hintereinander abläuft. Insbesondere ein Wechsel des landwirtschaftlichen Szenarios kann nahtlos erfolgen.

Es kann vorgesehen sein, dass das vorschlagsgemäße Verfahren und/oder die augmented reality-Routine automatisch und/oder vom Benutzer B initiiert werden. Beispielsweise kann sich der Benutzer B mit dem mobilen Gerät 5 in einem landwirtschaftlichen Zusammenhang bewegen, eine der augmented reality-Routine zugeordnete App auf dem mobilen Gerät 5 aktivieren, woraufhin dann automatisch ermittelt wird, in welchem der vordefinierten landwirtschaftlichen Szenarien sich das mobile Gerät 5 befindet. Der Begriff "landwirtschaftliches Szenario" betrifft dabei ganz allgemein unterschiedliche landwirtschaftliche Anwendungszusammenhänge, die hier und vorzugsweise gewählt sind aus der Gruppe umfassend eine Inspektion eines Tierbestands 3, eine Inspektion eines Feldbestands 9 und eine Feldbearbeitung, insbesondere mit einer landwirtschaftlichen Arbeitsmaschine 4.

Hier und vorzugsweise ist vorgesehen, dass den Anwendungsbausteinen A jeweils Identifikationsbedingungen zugeordnet sind. Diese Identifikationsbedingungen können dann dazu dienen, zu ermitteln, in welchem der vordefinierten landwirtschaftlichen Szenarien sich das mobile Gerät 5 befindet. Dabei kann vorgesehen sein, dass mehreren Anwendungsbausteinen A das gleiche landwirtschaftliche Szenario zugeordnet ist. In diesem Fall kommt der Ermittlung der Teilmenge der in der Datenbank D zu dem ermittelten landwirtschaftlichen Szenario gespeicherten Menge von Zusatzinformationen Z eine erhöhte Bedeutung zu, da die Menge an potentiell in dem landwirtschaftlichen Szenario anzeigbaren Zusatzinformationen Z durch die mehreren Anwendungsbausteine A erhöht ist. Es kann genauso vorteilhaft jedoch auch vorgesehen sein, dass jedem Anwendungsbaustein A genau ein landwirtschaftliches Szenario zugeordnet ist und/oder jedes landwirtschaftliche Szenario genau einem Anwendungsbaustein A zugeordnet ist.

Von der Steueranordnung 1 werden hier und vorzugsweise dem mobilen Gerät 5 zugeordnete Identifikationsinformationen ermittelt und mit den Identifikationsbedingungen abgeglichen. Basierend auf dem Ergebnis des Abgleichs wird von der Steueranordnung 1 ermittelt, in welchem der landwirtschaftlichen Szenarien sich das mobile Gerät 5 befindet.

Es kann dabei vorgesehen sein, dass beispielsweise die Identifikationsbedingungen mehrerer Anwendungsbausteine A erfüllt sind, denen jeweils eine kleine Menge an landwirtschaftlichen Szenarien zugeordnet ist, womit dann aus der Schnittmenge der landwirtschaftlichen Szenarien ermittelt werden kann, in welchem der landwirtschaftlichen Szenarien sich das mobile Gerät 5 befindet. Dabei ist auch nicht ausgeschlossen, dass sich das mobile Gerät 5 gleichzeitig in mehreren landwirtschaftlichen Szenarien befindet.

Hier und vorzugsweise umfassen die Identifikationsbedingungen das Vorhandensein von Identifikations-Objekten 10 der realen Welt. Von der Steueranordnung 1 können dann in der Umgebung des mobilen Geräts 5 vorhandene Objekte der realen Welt als Identifikationsinformationen ermittelt werden. Diese Identifikations-Objekte 10 können reale Pflanzen, Tiere, Maschinen, Gebäude und andere sein.

Es ist grundsätzlich bekannt, in einem augmented reality-Zusammenhang diverse Objekte der realen Welt in dem Abbild der Realität R mittels Bilderkennung zu erkennen. Je größer die Menge an Anwendungsbausteinen A im vorschlagsgemäßen Verfahren ist, desto aufwändiger wird jedoch die Identifikation des landwirtschaftlichen Szenarios. Ab einer gewissen Menge an Anwendungsbausteinen A kann es sogar unmöglich sein, mit der gegebenen Rechenleistung in annehmbarer Zeit nur über Bilderkennung das landwirtschaftliche Szenario zu ermitteln. Im landwirtschaftlichen Zusammenhang gibt es jedoch eine nicht unerhebliche, jedoch überschaubare, Menge an Objekten, die in der Lage sind, Funkverbindungen 11 aufzubauen. Diese Objekte sind über die verschiedenen landwirtschaftlichen Szenarien verteilt und können daher als Identifikations-Objekte 10 dienen. Es ist daher hier und vorzugsweise so, dass das mobile Gerät 5 über eine Funkverbindung 11 mit zumindest einem Objekt 4, 12, 13, 14 der realen Welt verbunden ist. Dieses Objekt der realen Welt ist vorzugsweise eine landwirtschaftliche Arbeitsmaschine 4 und/oder ein WLAN-Router 12 und/oder ein NFC-Tag 13 und/oder ein RFID-Tag 14. Hier und vorzugsweise wird von der Steueranordnung 1 auf Basis der Funkverbindung 11 auf das Vorhandensein des verbundenen Objekts 4, 12, 13, 14 als Identifikationsinformation geschlossen.

Es kann auch vorgesehen sein, dass von dem verbundenen Objekt 4, 12, 13, 14 der realen Welt über die Funkverbindung 11 weitere Identifikationsinformationen an das mobile Gerät 5 übertragen werden. Bei diesen weiteren Identifikationsinformationen kann es sich insbesondere um objektspezifische Daten des verbundenen Objekts 4, 12, 13, 14 und/oder Sensordaten von Sensoren 15 des verbundenen Objekts 4, 12, 13, 14 handeln. Beispielsweise könnten von einer landwirtschaftlichen Arbeitsmaschine 4 Sensordaten übertragen werden, die anzeigen, dass die landwirtschaftliche Arbeitsmaschine 4 gerade ein Feld 2 aberntet. Daraus könnte von der Steueranordnung 1 ermittelt werden, dass sich das mobile Gerät 5 in einem landwirtschaftlichen Szenario bei der Ernte eines Feldes 2 befindet.

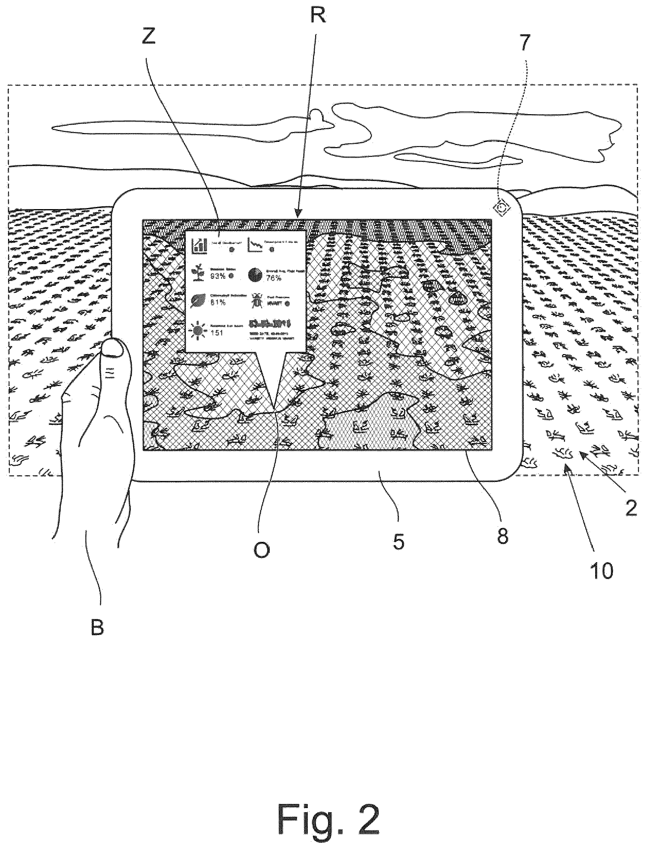

Im Folgenden werden anhand der Figuren 2 bis 4 drei bevorzugte landwirtschaftliche Szenarien erläutert.

Es kann wie in Fig. 2 dargestellt, vorgesehen sein, dass ein landwirtschaftliches Szenario "Feldinspektion" eine Inspektion eines landwirtschaftlichen Schlags ist. Vorzugsweise werden dann Felddaten, insbesondere Fruchtdaten und/oder Bodendaten und/oder Feldentwicklungsdaten und/oder Feldentwicklungserwartungsdaten und/oder Biomassedaten und/oder Pflanzengesundheitsdaten und/oder Chlorophyll-Sättigungsdaten und/oder Sonnenstunden und/oder Schädlingsbefalldaten, als Zusatzinformationen Z angezeigt. Die Felddaten sind hier und vorzugsweise positionsabhängig. So können beispielsweise die Daten zum Schädlingsbefall an verschiedenen Positionen des Felds 2 sehr unterschiedlich sein und entsprechend unterschiedlich angezeigt werden.

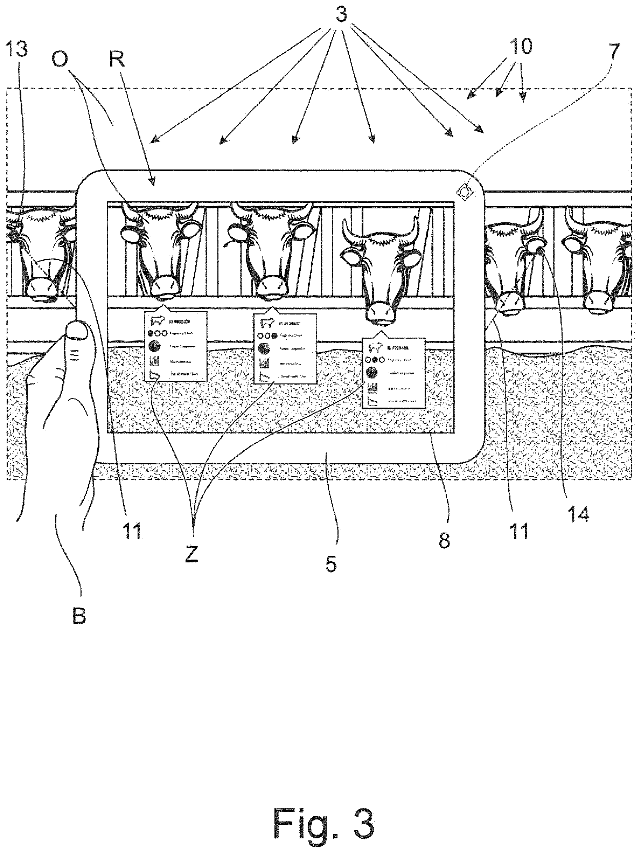

Hier und vorzugsweise ist zusätzlich oder alternativ ein weiteres, in Fig. 3 dargestelltes, landwirtschaftliches Szenario "Tierbestandsinspektion" eine Inspektion eines Tierbestands 3. Dabei werden vorzugsweise individuelle Daten eines spezifischen Tieres, insbesondere eine Tier-ID und/oder Schwangerschaftsdaten und/oder Futter-Zusammensetzungsdaten und/oder Milch-Leistungsdaten und/oder Tiergesundheitsdaten, als Zusatzinformationen Z angezeigt. So kann dem Benutzer B ermöglicht werden, alle relevanten Daten direkt bei der Pflege eines Tieres abzurufen.

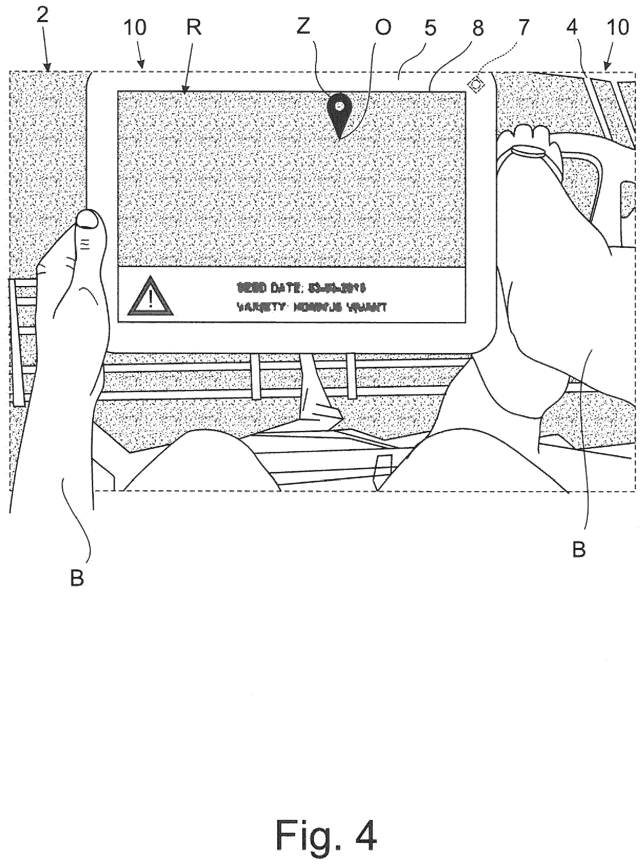

Weiter zusätzlich oder alternativ kann ein landwirtschaftliches Szenario "Feldbearbeitung" eine Feldbearbeitung mit einer landwirtschaftlichen Arbeitsmaschine 4 sein. Dies ist in Fig. 4 dargestellt. Dabei werden vorzugsweise Felddaten, insbesondere Fruchtdaten und/oder Bodendaten, insbesondere eine Bodenfeuchte, und/oder Maschinendaten der landwirtschaftlichen Arbeitsmaschine 4, insbesondere eine Temperatur und/oder eine geplante Fahrtroute, als Zusatzinformationen Z angezeigt.

Wie bereits erwähnt wurde, gibt es verschiedene Möglichkeiten, mittels derer von der Steueranordnung 1 ermittelt werden kann, in welchem landwirtschaftlichen Szenario sich das mobile Gerät 5 befindet. Es kann daher vorgesehen sein, dass das mobile Gerät 5 über eine Funkverbindung 11, insbesondere WLAN oder Bluetooth, mit der landwirtschaftlichen Arbeitsmaschine 4 verbunden ist und die Steueranordnung 1 auf Basis der Funkverbindung 11 ermittelt, das sich das mobile Gerät 5 im landwirtschaftlichen Szenario "Feldbearbeitung" befindet.

Wie sich anhand der Unterschiedlichkeit der geschilderten landwirtschaftlichen Szenarien ergibt, können dort auch unterschiedliche mobile Geräte 5 vorhanden sein. Entsprechend ist es vorzugsweise so, dass das mobile Gerät 5 ein Smartphone oder ein Tablet oder ein Mobiltelefon oder ein Head-up Display einer landwirtschaftlichen Arbeitsmaschine 4 oder eine Datenbrille ist. Es kann ganz allgemein vorgesehen sein, dass die Kamera 7 in das mobile Gerät 5 eingebaut ist.

Hier und vorzugsweise wird das mobile Gerät 5 während der augmented reality-Routine von dem Benutzer B bewegt. Dabei kann sich das angezeigte Abbild der Realität R mit dem abgebildeten Objekt O der realen Welt ändern, womit sich gleichzeitig auch die angezeigten Zusatzinformationen Z ändern.

Weitere Vorteile des vorschlagsgemäßen Verfahrens ergeben sich, wenn der Benutzer B Eingaben an dem mobilen Gerät 5 tätigen kann, von der Steueranordnung 1 die Eingaben einem abgebildeten Objekt O zugeordnet werden, auf Basis der Eingaben die angezeigten Zusatzinformationen Z von der Steueranordnung 1 modifiziert werden und die modifizierten Zusatzinformationen Z in der Datenbank D abgespeichert werden. So wird es dem Benutzer B ermöglicht, die Zusatzinformationen Z nicht nur vor Ort abzurufen, sondern auch Dokumentationen vor Ort vorzunehmen. Es kann vorgesehen sein, dass die oben erwähnten Felddaten positionsabhängig durch Eingaben des Benutzers B am mobilen Gerät 5 modifiziert werden. So kann der Benutzer B während der Inspektion des landwirtschaftlichen Schlags beispielsweise direkt einen Schädlingsbefall positionsabhängig dokumentieren.

Zusätzlich oder alternativ kann vorgesehen sein, dass die individuellen Daten des spezifischen Tieres durch Eingaben des Benutzers B am mobilen Gerät 5 modifiziert werden. So kann der Benutzer B beispielsweise eines Fütterung eines Tieres direkt tierspezifisch dokumentieren.

Insbesondere zu Beginn des vorschlagsgemäßen Verfahrens kann vorgesehen sein, dass der Benutzer B die Kamera 7 auf ein spezifisches Feld 2 richtet und die Steueranordnung 1 das Vorhandensein eines allgemeinen Feldes 2 als Identifikationsinformationen ermittelt. Es ist hier also möglich, dass von der Steueranordnung 1 aus dem Abbild der Realität R lediglich entnommen werden kann, dass die Kamera 7 auf ein Feld 2 gerichtet ist, jedoch nicht oder nicht direkt ermittelt werden kann, welches einer Menge an potentiellen Feldern 2 abgebildet ist.

Es kann dann zusätzlich oder alternativ vorgesehen sein, dass von der Steueranordnung 1, insbesondere über GPS, Positionsinformationen des mobilen Geräts 5 als Identifikationsinformationen ermittelt werden.

Vorzugsweise wird von der Steueranordnung 1 auf Basis der Identifikationsinformationen, insbesondere auf Basis des Vorhandenseins eines allgemeinen Feldes 2 und/oder von feldspezifischen Identifikations-Objekten 10, beispielsweise dem Feldbestand 9, und/oder auf Basis der Positionsinformationen, ermittelt, dass sich das mobile Gerät 5 im landwirtschaftlichen Szenario "Feldinspektion" befindet. Dies ist in Fig. 2 verdeutlicht.

Weiter vorzugsweise wird von der Steueranordnung 1 auf Basis der Identifikationsinformationen das spezifische Feld 2 aus einer in der Datenbank D gespeicherten Menge vordefinierter spezifischer Felder 2 ermittelt. Abhängig von dem spezifischen Feld 2 kann von der Steueranordnung 1 dann die Menge potentieller in der Datenbank D gespeicherter Zusatzinformationen Z ermittelt werden.

So kann im Zusammenspiel von Kamera 7 und GPS ermittelt werden, an welchem spezifischen Feld 2 der Benutzer B mit dem mobilen Gerät 5 steht. Dadurch können dem Benutzer B Zusatzinformationen Z angezeigt werden, die dieses spezifische Feld 2 betreffen. Gerade bei landwirtschaftlichen Anwendungen erlaubt die Nutzung von GPS konkrete Rückschlüsse auf das landwirtschaftliche Szenario, in dem sich das mobile Gerät 5 befindet. Sofern das spezifische Feld 2 in der in der Datenbank D gespeicherten Menge vordefinierter spezifischer Felder 2 vorhanden ist und die Position des mobilen Geräts 5 mittels GPS ermittelt wird, können die potentiellen landwirtschaftlichen Szenarien sofort auf jene eingegrenzt werden, die mit diesem spezifischen Feld 2 in Zusammenhang stehen.

Da sich landwirtschaftliche Szenarien jedoch nicht nur auf Felder 2 erstrecken, kann vorgesehen sein, dass der Benutzer B die Kamera 7 auf zumindest ein spezifisches Tier richtet und von der Steueranordnung 1 das Vorhandensein eines allgemeinen Tieres als Identifikationsinformation ermittelt wird. Da analog zum spezifischen Feld 2 das spezifische Tier möglicherweise nicht ohne gro-ßen Aufwand mittels Bilderkennung identifiziert werden kann, kann vorgesehen sein, dass das mobile Gerät 5 über eine Funkverbindung 11, insbesondere über RFID, mit einem dem Tier zugeordneten verbundenen Objekt 13, 14 verbunden ist. Dies ist möglich, da Tiere eines landwirtschaftlichen Tierbestands 3 häufig mit RFID-Tags 14 gekennzeichnet sind.

Vorzugsweise wird auf Basis der Identifikationsinformationen, insbesondere dem Vorhandensein des allgemeinen Tieres und/oder dem Vorhandensein des verbundenen Objekts 13, 14, und/oder der Funkverbindung 11 von der Steueranordnung 1 ermittelt, dass sich das mobile Gerät 5 im, in Fig. 3 dargestellten, landwirtschaftlichen Szenario "Tierbestandsinspektion" befindet. Weiter vorzugsweise wird von der Steueranordnung 1, insbesondere auf Basis der Funkverbindung 11, das spezifische Tier aus einer in der Datenbank D gespeicherten Menge vordefinierter spezifischer Tiere ermittelt und abhängig von dem spezifischen Tier wird von der Steueranordnung 1 vorzugsweise die Menge potentieller in der Datenbank D gespeicherter Zusatzinformationen Z ermittelt.

Bei dem in Fig. 4 dargestellten landwirtschaftlichen Szenario "Feldbearbeitung" kann es sich vorzugsweise um ein Pflügen eines Feldes 2, ein Aussäen einer Saat, ein Abernten des Feldbestandes 9 oder Ähnliches handeln. Dabei sind für den Benutzer B sowohl Zusatzinformationen Z über das Feld 2 von Bedeutung, als auch Zusatzinformationen Z, die der landwirtschaftlichen Arbeitsmaschine 4 zugeordnet sind. Auch Zusatzinformationen, die die konkrete Tätigkeit betreffen, können hier relevant sein.

Wie bereits angesprochen, kann sich das mobile Gerät 5 in mehreren landwirtschaftlichen Szenarien gleichzeitig befinden. Bei den obigen landwirtschaftlichen Szenarien kann daher vorgesehen sein, dass von der Steueranordnung 1 ermittelt wird, dass sich das mobile Gerät 5 in dem landwirtschaftlichen Szenario "Feldbearbeitung" befindet und daraus von der Steueranordnung 1 ermittelt wird, dass sich das mobile Gerät 5 auch im landwirtschaftlichen Szenario "Feldinspektion" befindet. Es kann dabei jedoch vorgesehen sein, dass nicht alle der Zusatzinformationen Z, die in dem landwirtschaftlichen Szenario "Feldinspektion" angezeigt würden, wenn sich das mobile Gerät 5 nicht gleichzeitig im landwirtschaftlichen Szenario "Feldbearbeitung" befindet, auch angezeigt werden, wenn sich das mobile Gerät 5 gleichzeitig im landwirtschaftlichen Szenario "Feldbearbeitung" befindet.

Ganz allgemein umfassen die Anwendungsbausteine A vorzugsweise zumindest einen dem landwirtschaftlichen Szenario "Feldinspektion" zugeordneten Anwendungsbaustein A und/oder einen dem landwirtschaftlichen Szenario "Tierbestandsinspektion" zugeordneten Anwendungsbaustein A und/oder einen dem landwirtschaftlichen Szenario "Feldbearbeitung" zugeordneten Anwendungsbaustein A.

Aufgrund der hohen Flexibilität des vorschlagsgemäßen Verfahrens kann vorgesehen sein, dass die Anwendungsbausteine A von verschiedenen Herstellern bereitgestellt werden. Das heißt, es wird möglich, mittels einer Serveranwendung 6 dem Benutzer B viele unterschiedliche Zusatzinformationen Z zur Verfügung zu stellen.

Zusätzlich oder alternativ kann vorgesehen sein, dass der Benutzer B viele verschiedene Anwendungsbausteine A vorauswählen kann und dass die vordefinierten landwirtschaftlichen Szenarien der vorausgewählten Anwendungsbausteine A zugeordnet sind. So kann es vorteilhaft sein, wenn von der Steueranordnung 1 keine landwirtschaftlichen Szenarien erkannt werden, zu denen keine vorausgewählten Anwendungsbausteine A existieren, da dann möglicherweise auch keine anzeigbaren Zusatzinformationen Z existieren.

Ganz allgemein ist es auch vorzugsweise so, dass zumindest zwei landwirtschaftliche Szenarien vorgesehen sind, die nur an verschiedenen Orten möglich sind. Vorzugsweise betrifft zumindest ein landwirtschaftliches Szenario einen Hof und ein anderes landwirtschaftliches Szenario ein Feld.

Nach einer weiteren Lehre, der eigenständige Bedeutung zukommt, wird ein mobiles Gerät 5 beansprucht, das zur Verwendung in einem vorschlagsgemäßen Verfahren ausgestaltet ist. Auf alle Ausführungen zu dem vorschlagsgemäßen Verfahren darf verwiesen werden. Dabei kann das mobile Gerät 5 insbesondere eine App mit Zugangsinformationen aufweisen, die die Kommunikation mit der Serveranwendung 6 ermöglichen.

Nach einer weiteren Lehre, der ebenfalls eigenständige Bedeutung zukommt, wird eine Serveranwendung 6 beansprucht, die zur Verwendung in einem vorschlagsgemäßen Verfahren ausgestaltet ist. Auf alle Ausführungen zu dem vorschlagsgemäßen Verfahren darf verwiesen werden. Bei der Serveranwendung 6 kommt insbesondere einer Software getrennt von der Hardware eigenständige Bedeutung zu. Gleichzeitig kommt auch einer Hardware, die zur Verwendung in dem vorschlagsgemäßen Verfahren ausgestaltet ist, eigenständige Bedeutung zu.

Die vorschlagsgemäße Software kann dabei die Datenbank D umfassen oder zur Kommunikation mit einer Datenbank D ausgestaltet sein.

### Bezugszeichenliste

- 1: Steueranordnung
- 2: Feld
- 3: Tierbestand
- 4: landwirtschaftliche Arbeitsmaschine
- 5: mobiles Gerät
- 6: Serveranwendung
- 7: Kamera
- 8: Display
- 9: Feldbestand
- 10: Identifikations-Objekte
- 11: Funkverbindung
- 12: WLAN-Router
- 13: NFC-Tag
- 14: RFID-Tag
- 15: Sensoren
- B: Benutzer
- D: Datenbank
- R: Abbild der Realität
- Z: Zusatzinformationen
- O: abgebildetes Objekt
- A: Anwendungsbaustein

## Patentansprüche

1. Verfahren zur Unterstützung eines Benutzers (B) bei einer landwirtschaftlichen Tätigkeit mittels einer Steueranordnung (1),
wobei die Steueranordnung (1) zumindest ein mobiles Gerät (5) und eine mit dem mobilen Gerät (5) kommunizierende Serveranwendung (6), wobei die Serveranwendung einen oder mehrere Server umfasst, auf denen ein entsprechendes Computerprogramm ausgeführt wird und auf denen eine Datenbank (D) abgespeichert ist, (D) aufweist,
wobei das mobile Gerät (5) eine Kamera (7) und ein Display (8) aufweist,
wobei von der Steueranordnung (1) eine augmented reality-Routine ausgeführt wird,
wobei in der augmented reality-Routine auf dem Display (8) des mobilen Geräts (5) ein von der Kamera (7) erzeugtes Abbild der Realität (R) und mindestens eine in der Datenbank (D) gespeicherte Zusatzinformation (Z), die einem landwirtschaftlichen Szenario zugeordnet ist, visuell überlagert angezeigt werden und
wobei die jeweilige Zusatzinformation (Z) mindestens einem in dem Abbild der Realität (R) abgebildeten Objekt (O) der realen Welt zugeordnet ist,
**dadurch gekennzeichnet,**
**dass** die Serveranwendung (6) mehrere Anwendungsbausteine (A), denen jeweils ein vordefiniertes landwirtschaftliches Szenario zugeordnet ist, umfasst und in der Datenbank (D) zu jedem landwirtschaftlichen Szenario eine Menge von Zusatzinformationen (Z) gespeichert ist,
**dass** von der Steueranordnung (1) automatisch ermittelt wird, in welchem der vordefinierten landwirtschaftlichen Szenarien sich das mobile Gerät (5) befindet,
**dass** von der Steueranordnung (1) abhängig von dem ermittelten landwirtschaftlichen Szenario die Menge in der Datenbank (D) gespeicherter Zusatzinformationen (Z) ermittelt wird und
**dass** abhängig von mindestens einem in dem Abbild der Realität (R) abgebildeten Objekt (O) eine Teilmenge der in der Datenbank (D) zu dem ermittelten landwirtschaftlichen Szenario gespeicherten Menge von Zusatzinformationen (Z) auf dem Display (8) angezeigt wird.

2. Verfahren nach Anspruch 1, **dadurch gekennzeichnet, dass** den Anwendungsbausteinen (A) jeweils Identifikationsbedingungen zugeordnet sind, dass die Steueranordnung (1) dem mobilen Gerät (5) zugeordnete Identifikationsinformationen ermittelt und mit den Identifikationsbedingungen abgleicht und dass von der Steueranordnung (1) basierend auf dem Ergebnis des Abgleichs ermittelt wird, in welchem der landwirtschaftlichen Szenarien sich das mobile Gerät (5) befindet.

3. Verfahren nach Anspruch 2, **dadurch gekennzeichnet, dass** die Identifikationsbedingungen das Vorhandensein von Identifikations-Objekten (10) der realen Welt umfassen und dass von der Steueranordnung (1) in der Umgebung des mobilen Geräts (5) vorhandene Objekte der realen Welt als Identifikationsinformationen ermittelt werden.

4. Verfahren nach Anspruch 2 oder 3, **dadurch gekennzeichnet, dass** das mobile Gerät (5) über eine Funkverbindung (11) mit zumindest einem Objekt der realen Welt, vorzugsweise einer landwirtschaftlichen Arbeitsmaschine (4) und/oder einem WLAN-Router (12) und/oder einem NFC-Tag (13) und/oder einem RFID-Tag (14), verbunden ist, vorzugsweise, dass von der Steueranordnung (1) auf Basis der Funkverbindung (11) auf das Vorhandensein des verbundenen Objekts (4, 12, 13, 14) als Identifikationsinformation geschlossen wird, weiter vorzugsweise, dass von dem verbundenen Objekt (4, 12, 13, 14) der realen Welt über die Funkverbindung (11) weitere Identifikationsinformationen, insbesondere objektspezifische Daten des verbundenen Objekts (4, 12, 13, 14) und/oder Sensordaten von Sensoren (15) des verbundenen Objekts (4, 12, 13, 14), an das mobile Gerät (5) übertragen werden.

5. Verfahren nach einem der vorhergehenden Ansprüche, **dadurch gekennzeichnet, dass** ein landwirtschaftliches Szenario "Feldinspektion" eine Inspektion eines landwirtschaftlichen Schlags ist, vorzugsweise, dass Felddaten, insbesondere Fruchtdaten und/oder Bodendaten und/oder Feldentwicklungsdaten und/oder Feldentwicklungserwartungsdaten und/oder Biomassedaten und/oder Pflanzengesundheitsdaten und/oder Chlorophyll-Sättigungsdaten und/oder Sonnenstunden und/oder Schädlingsbefalldaten, als Zusatzinformationen (Z) angezeigt werden, weiter vorzugsweise, dass die Felddaten positionsabhängig sind.

6. Verfahren nach einem der vorhergehenden Ansprüche, **dadurch gekennzeichnet, dass** ein landwirtschaftliches Szenario "Tierbestandsinspektion" eine Inspektion eines Tierbestands (3) ist, vorzugsweise, dass individuelle Daten eines spezifischen Tieres, insbesondere eine Tier-ID und/oder Schwangerschaftsdaten und/oder Futter-Zusammensetzungsdaten und/oder Milch-Leistungsdaten und/oder Tiergesundheitsdaten, als Zusatzinformationen (Z) angezeigt werden.

7. Verfahren nach einem der vorhergehenden Ansprüche, **dadurch gekennzeichnet, dass** ein landwirtschaftliches Szenario "Feldbearbeitung" eine Feldbearbeitung mit einer landwirtschaftlichen Arbeitsmaschine (4) ist,
vorzugsweise, dass Felddaten, insbesondere Fruchtdaten und/oder Bodendaten, insbesondere eine Bodenfeuchte, und/oder Maschinendaten der landwirtschaftlichen Arbeitsmaschine, insbesondere eine Temperatur und/oder eine geplante Fahrtroute, als Zusatzinformationen (Z) angezeigt werden, und/oder,
dass das mobile Gerät (5) über eine Funkverbindung (11), insbesondere WLAN oder Bluetooth, mit der landwirtschaftlichen Arbeitsmaschine (4) verbunden ist und von der Steueranordnung (1) auf Basis der Funkverbindung (11) ermittelt wird, dass sich das mobile Gerät (5) im landwirtschaftlichen Szenario "Feldbearbeitung" befindet.

8. Verfahren nach einem der vorhergehenden Ansprüche, **dadurch gekennzeichnet, dass** das mobile Gerät (5) ein Smartphone oder ein Tablet oder ein Mobiltelefon oder ein Head-up Display einer landwirtschaftlichen Arbeitsmaschine (4) oder eine Datenbrille ist, und/oder, dass die Kamera (7) in das mobile Gerät (5) eingebaut ist.

9. Verfahren nach einem der vorhergehenden Ansprüche, **dadurch gekennzeichnet, dass** der Benutzer (B) Eingaben an dem mobilen Gerät (5) tätigt, dass von der Steueranordnung (1) die Eingaben einem abgebildeten Objekt (O) zugeordnet werden, auf Basis der Eingaben die angezeigten Zusatzinformationen (Z) von der Steueranordnung (1) modifiziert werden und die modifizierten Zusatzinformationen (Z) in der Datenbank (D) abgespeichert werden, vorzugsweise, dass die Felddaten positionsabhängig durch Eingaben des Benutzers (B) am mobilen Gerät (5) modifiziert werden, und/oder, dass die individuellen Daten des spezifischen Tieres durch Eingaben des Benutzers (B) am mobilen Gerät (5) modifiziert werden.

10. Verfahren nach einem der vorhergehenden Ansprüche, **dadurch gekennzeichnet, dass** der Benutzer (B) die Kamera (7) auf ein spezifisches Feld (2) richtet und von der Steueranordnung (1) das Vorhandensein eines allgemeinen Feldes (2) als Identifikationsinformationen ermittelt wird, und/oder, dass von der Steueranordnung (1), insbesondere über GPS, Positionsinformationen des mobilen Geräts als Identifikationsinformationen ermittelt werden, vorzugsweise, dass auf Basis der Identifikationsinformationen von der Steueranordnung (1) ermittelt wird, dass sich das mobile Gerät (5) im landwirtschaftlichen Szenario "Feldinspektion" befindet, weiter vorzugsweise, dass von der Steueranordnung (1) auf Basis der Identifikationsinformationen das spezifische Feld (2) aus einer in der Datenbank (D) gespeicherten Menge vordefinierter spezifischer Felder (2) ermittelt wird und von der Steueranordnung (1) abhängig von dem spezifischen Feld (2) die Menge potentieller in der Datenbank (D) gespeicherter Zusatzinformationen ermittelt wird.

11. Verfahren nach einem der vorhergehenden Ansprüche, **dadurch gekennzeichnet, dass** der Benutzer (B) die Kamera auf zumindest ein spezifisches Tier richtet und von der Steueranordnung (1) das Vorhandensein eines allgemeinen Tieres als Identifikationsinformationen ermittelt wird und/oder das mobile Gerät (5) über eine Funkverbindung (11), insbesondere über RFID, mit einem dem Tier zugeordneten verbundenen Objekt (13, 14) verbunden ist, vorzugsweise, dass von der Steueranordnung (1) auf Basis der Identifikationsinformationen und/oder der Funkverbindung (11) ermittelt wird, dass sich das mobile Gerät (5) im landwirtschaftlichen Szenario "Tierbestandsinspektion" befindet, weiter vorzugsweise, dass von der Steueranordnung (1), insbesondere auf Basis der Funkverbindung (11), das spezifische Tier aus einer in der Datenbank (D) gespeicherten Menge vordefinierter spezifischer Tiere ermittelt wird und von der Steueranordnung (1) abhängig von dem spezifischen Tier die Menge potentieller in der Datenbank (D) gespeicherter Zusatzinformationen (Z) ermittelt wird.

12. Verfahren nach einem der vorhergehenden Ansprüche, **dadurch gekennzeichnet, dass** von der Steueranordnung (1) ermittelt wird, dass sich das mobile Gerät (5) im landwirtschaftlichen Szenario "Feldbearbeitung" befindet und von der Steueranordnung (1) daraus ermittelt wird, dass sich das mobile Gerät (5) auch im landwirtschaftlichen Szenario "Feldinspektion" befindet.

13. Verfahren nach einem der vorhergehenden Ansprüche, **dadurch gekennzeichnet, dass** die Anwendungsbausteine (A) zumindest einen dem landwirtschaftlichen Szenario "Feldinspektion" zugeordneten Anwendungsbaustein (A) und/oder einen dem landwirtschaftlichen Szenario "Tierbestandsinspektion" zugeordneten Anwendungsbaustein (A) und/oder einen dem landwirtschaftlichen Szenario "Feldbearbeitung" zugeordneten Anwendungsbaustein (A) umfassen, und/oder, dass die Anwendungsbausteine (A) von verschiedenen Hersteller bereitgestellt werden und/oder, dass der Benutzer (B) verschiedene Anwendungsbausteine (A) vorauswählen kann und dass die vordefinierten landwirtschaftlichen Szenarien den vorausgewählten Anwendungsbausteinen (A) zugeordnet sind.

14. Mobiles Gerät (5) ausgestaltet zur Verwendung in einem Verfahren nach einem der Ansprüche 1 bis 13, wobei das mobile Gerät (5) eine Kamera (7) und ein Display (8) aufweist,
wobei das mobile Gerät (5) so ausgestaltet ist, dass eine Serveranwendung mit dem mobilen Gerät kommuniziert, wobei die Serveranwendung einen oder mehrere Server umfasst, auf denen ein entsprechendes Computerprogramm ausgeführt wird und auf denen eine Datenbank (D) abgespeichert ist,
wobei in der augmented reality-Routine auf dem Display (8) des mobilen Geräts (5) ein von der Kamera (7) erzeugtes Abbild der Realität (R) und mindestens eine in der Datenbank (D) gespeicherte Zusatzinformation (Z), die einem landwirtschaftlichen Szenario zugeordnet ist, visuell überlagert angezeigt werden und
wobei die jeweilige Zusatzinformation (Z) mindestens einem in dem Abbild der Realität (R) abgebildeten Objekt (O) der realen Welt zugeordnet ist.

15. Serveranwendung (6) mit einer Datenbank (D) ausgestaltet zur Verwendung in einem Verfahren nach einem der Ansprüche 1 bis 13, wobei die Serveranwendung einen oder mehrere Server umfasst, auf denen ein entsprechendes Computerprogramm ausgeführt wird und auf denen die Datenbank (D) abgespeichert ist, wobei die Serveranwendung (6) mit einem mobilen Gerät (5) kommuniziert, wobei die Serveranwendung (6) mehrere Anwendungsbausteine (A), denen jeweils ein vordefiniertes landwirtschaftliches Szenario zugeordnet ist, umfasst und in der Datenbank (D) zu jedem landwirtschaftlichen Szenario eine Menge von Zusatzinformationen (Z) gespeichert ist,
dass von der Steueranordnung (1) automatisch ermittelt wird, in welchem der vordefinierten landwirtschaftlichen Szenarien sich das mobile Gerät (5) befindet.

## Claims

1. method for assisting a user (B) in an agricultural activity by means of a control arrangement (1),
wherein the control arrangement (1) comprises at least one mobile device (5) and a server application (6) communicating with the mobile device (5), wherein the server application comprises one or more servers on which a corresponding computer program is executed and on which a database (D) is stored,
wherein the mobile device (5) has a camera (7) and a display (8),
wherein an augmented reality routine is executed by the control arrangement (1), wherein in the augmented reality routine an image of reality (R) generated by the camera (7) and at least one piece of additional information (Z) stored in the database (D), which is associated with an agricultural scenario, are displayed visually superimposed on the display (8) of the mobile device (5), and
wherein the respective additional information (Z) is assigned to at least one object (O) of the real world depicted in the image of reality (R),
**characterised in that**
**in that** the server application (6) comprises a plurality of application modules (A), each of which is assigned a predefined agricultural scenario, and a quantity of additional information (Z) is stored in the database (D) for each agricultural scenario,
that the control arrangement (1) automatically determines in which of the predefined agricultural scenarios the mobile device (5) is located,
**in that** the amount of additional information (Z) stored in the database (D) is determined by the control arrangement (1) as a function of the determined agricultural scenario, and **in that**, depending on at least one object (O) depicted in the image of reality (R), a subset of the amount of additional information (Z) stored in the database (D) for the determined agricultural scenario is shown on the display (8).

2. method according to claim 1, **characterised in that** the application modules (A) are each assigned identification conditions, **in that** the control arrangement (1) determines identification information assigned to the mobile device (5) and compares it with the identification conditions, and **in that** the control arrangement (1) determines in which of the agricultural scenarios the mobile device (5) is located based on the result of the comparison.

3. method according to claim 2, **characterised in that** the identification conditions comprise the presence of identification objects (10) of the real world and that objects of the real world present in the environment of the mobile device (5) are determined as identification information by the control arrangement (1).

4. Method according to claim 2 or 3, **characterised in that** the mobile device (5) is connected via a radio link (11) to at least one object in the real world, preferably an agricultural machine (4) and/or a WLAN router (12) and/or an NFC tag (13) and/or an RFID tag (14), preferably **in that** the control arrangement (1) uses the radio link (11) to infer the presence of the connected object (4, 12, 13, 14) as identification information on the basis of the radio link (11), and further preferably that further identification information, in particular object-specific data of the connected object (4, 12, 13, 14) and/or sensor data from sensors (15) of the connected object (4, 12, 13, 14), is transmitted to the mobile device (5) by the connected object (4, 12, 13, 14) in the real world via the radio link (11).

5. method according to one of the preceding claims, **characterised in that** an agricultural scenario "field inspection" is an inspection of an agricultural field, preferably **in that** field data, in particular crop data and/or soil data and/or field development data and/or field development expectation data and/or biomass data and/or plant health data and/or chlorophyll saturation data and/or sunshine hours and/or pest infestation data, are displayed as additional information (Z), further preferably **in that** the field data are position-dependent.

6. method according to one of the preceding claims, **characterised in that** an agricultural scenario "livestock inspection" is an inspection of a livestock (3), preferably that individual data of a specific animal, in particular an animal ID and/or pregnancy data and/or feed composition data and/or milk performance data and/or animal health data, are displayed as additional information (Z).

7. method according to one of the preceding claims, **characterised in that** an agricultural scenario "field cultivation" is a field cultivation with an agricultural working machine (4), preferably that field data, in particular crop data and/or soil data, in particular soil moisture, and/or machine data of the agricultural machine, in particular a temperature and/or a planned route, are displayed as additional information (Z), and/or,
**in that** the mobile device (5) is connected to the agricultural machine (4) via a radio link (11), in particular WLAN or Bluetooth, and the control arrangement (1) determines on the basis of the radio link (11) that the mobile device (5) is in the agricultural scenario "field cultivation".

8. method according to one of the preceding claims, **characterised in that** the mobile device (5) is a smartphone or a tablet or a mobile telephone or a head-up display of an agricultural machine (4) or data goggles, and/or **in that** the camera (7) is built into the mobile device (5).

9. Method according to one of the preceding claims, **characterised in that** the user (B) makes inputs on the mobile device (5), **in that** the inputs are assigned to an imaged object (O) by the control arrangement (1), the displayed additional information (Z) is modified by the control arrangement (1) on the basis of the inputs and the modified additional information (Z) is stored in the database (D), preferably that the field data are modified depending on the position by inputs of the user (B) on the mobile device (5), and/or that the individual data of the specific animal are modified by inputs of the user (B) on the mobile device (5).

10. Method according to one of the preceding claims, **characterised in that** the user (B) points the camera (7) at a specific field (2) and the presence of a general field (2) is determined by the control arrangement (1) as identification information, and/or **in that** position information of the mobile device is determined by the control arrangement (1), in particular via GPS, as identification information, preferably **in that** the control arrangement (1) determines the specific field (2) on the basis of the identification information, that the mobile device (5) is located in the agricultural scenario "field inspection", further preferably that the specific field (2) is determined by the control arrangement (1) on the basis of the identification information from a set of predefined specific fields (2) stored in the database (D) and the set of potential additional information stored in the database (D) is determined by the control arrangement (1) as a function of the specific field (2).

11. Method according to one of the preceding claims, **characterised in that** the user (B) points the camera at at least one specific animal and the presence of a general animal is determined by the control arrangement (1) as identification information and/or the mobile device (5) is connected via a radio link (11), in particular via RFID, to a connected object (13, 14) associated with the animal, preferably **in that** the control arrangement (1) determines on the basis of the identification information and/or the radio link (11), that the mobile device (5) is in the agricultural scenario "livestock inspection", further preferably that the specific animal is determined by the control arrangement (1), in particular on the basis of the radio connection (11), from a quantity of predefined specific animals stored in the database (D) and the quantity of potential additional information (Z) stored in the database (D) is determined by the control arrangement (1) as a function of the specific animal.

12. method according to one of the preceding claims, **characterised in that** it is determined by the control arrangement (1) that the mobile device (5) is in the agricultural scenario "field processing" and it is determined from this by the control arrangement (1) that the mobile device (5) is also in the agricultural scenario "field inspection".

13. Method according to one of the preceding claims, **characterised in that** the application modules (A) comprise at least one application module (A) assigned to the agricultural scenario "field inspection" and/or one application module (A) assigned to the agricultural scenario "livestock inspection" and/or one application module (A) assigned to the agricultural scenario "field processing", and/or that the application modules (A) are provided by different manufacturers and/or that the user (B) can preselect different application modules (A) and that the predefined agricultural scenarios are assigned to the preselected application modules (A).

14. mobile device (5) designed for use in a method according to one of claims 1 to 13,
wherein the mobile device (5) comprises a camera (7) and a display (8),
wherein the mobile device (5) is designed such that a server application communicates with the mobile device, wherein the server application comprises one or more servers on which a corresponding computer program is executed and on which a database (D) is stored, wherein in the augmented reality routine an image of reality (R) generated by the camera (7) and at least one additional information item (Z) stored in the database (D), which is associated with an agricultural scenario, are displayed visually superimposed on the display (8) of the mobile device (5), and
wherein the respective additional information (Z) is assigned to at least one object (O) of the real world depicted in the image of reality (R).

15. A server application (6) with a database (D) designed for use in a method according to any one of claims 1 to 13, wherein the server application comprises one or more servers on which a corresponding computer program is executed and on which a database (D) is stored, wherein the server application (6) communicates with a mobile device (5), wherein the server application (6) comprises several application modules (A), to each of which a predefined agricultural scenario is assigned, and a quantity of additional information (Z) is stored in the database (D) for each agricultural scenario,
that the control arrangement (1) automatically determines in which of the predefined agricultural scenarios the mobile device (5) is located.

## Revendications

1. Procédé d'assistance d'un utilisateur (B) dans une activité agricole au moyen d'un agencement de commande (1),
l'agencement de commande (1) comportant au moins un appareil mobile (5) et une application de serveur (6) communiquant avec l'appareil mobile (5), l'application de serveur incluant un ou plusieurs serveurs sur lesquels est exécuté un programme informatique correspondant et sur lesquels est stockée une banque de données (D),
l'appareil mobile (5) comportant une caméra (7) et un affichage (8), un programme de réalité augmentée étant exécuté par l'agencement de commande (1),
dans le programme de réalité augmentée, une reproduction de la réalité (R) générée par la caméra (7) et au moins une information supplémentaire (Z) stockée dans la banque de données (D) et associée à un scénario agricole étant affichées sur l'affichage (8) de l'appareil mobile (5) en étant superposées visuellement et
l'information supplémentaire respective (Z) étant associée au moins à un objet (0) du monde réel reproduit dans la reproduction de la réalité (R),
**caractérisé en ce que**
l'application de serveur (6) inclut plusieurs modules d'application (A) auxquels un scénario agricole prédéfini est respectivement associé, et une quantité d'informations supplémentaires (Z) relative à chaque scénario agricole est stockée dans la banque de données (D),
l'agencement de commande (1) détermine automatiquement dans lequel des scénarios agricoles prédéfinis se trouve l'appareil mobile (5),
en fonction du scénario agricole déterminé, l'agencement de commande (1) détermine la quantité d'informations supplémentaires (Z) stockées dans la banque de données (D) et
en fonction d'au moins un objet (0) reproduit dans la reproduction de la réalité (R), une quantité partielle de la quantité d'informations supplémentaires (Z) stockées dans la banque de données (D) pour le scénario agricole déterminé est affichée sur l'affichage (8).

2. Procédé selon la revendication 1, **caractérisé en ce qu'**aux modules d'application (A) sont respectivement associées des conditions d'identification, **en ce que** l'agencement de commande (1) détermine des informations d'identification associées à l'appareil mobile (5) et les compare avec les conditions d'identification, et **en ce que** l'agencement de commande (1) détermine, sur la base du résultat de la comparaison, dans lequel des scénarios agricoles se trouve l'appareil mobile (5).

3. Procédé selon la revendication 2, **caractérisé en ce que** les conditions d'identification incluent la présence d'objets d'identification (10) dans le monde réel et **en ce que** l'agencement de commande (1) détermine des objets du monde réel présents dans l'environnement de l'appareil mobile (5) en tant qu'informations d'identification.

4. Procédé selon la revendication 2 ou 3, **caractérisé en ce que** l'appareil mobile (5) est relié par l'intermédiaire d'une liaison radio (11) à au moins un objet du monde réel, préférentiellement à une machine de travail agricole (4) et/ou à un routeur WLAN (12) et/ou à une étiquette NFC (13) et/ou à une étiquette RFID (14), préférentiellement **en ce que** l'agencement de commande (1) conclut, sur la base de la liaison radio (11), à la présence de l'objet relié (4, 12, 13, 14), plus préférentiellement **en ce que** l'objet relié (4, 12, 13, 14) du monde réel transmet à l'appareil mobile (5), par l'intermédiaire de la liaison radio (11), d'autres informations d'identification, en particulier des données de l'objet relié (4, 12, 13, 14) spécifiques à l'objet et/ou des données de capteur de capteurs (15) de l'objet relié (4, 12, 13, 14).

5. Procédé selon une des revendications précédentes, **caractérisé en ce qu'**un scénario agricole « inspection de champ » est une inspection d'une parcelle agricole, préférentiellement **en ce que** des données de champ, en particulier des données de produit et/ou des données de sol et/ou des données de développement de champ et/ou des données escomptées de développement de champ et/ou des données de biomasse et/ou des données phytosanitaires et/ou des données de saturation en chlorophylle et/ou des heures de soleil et/ou des données d'infestation par des nuisibles, sont affichées en tant qu'informations supplémentaires (Z), plus préférentiellement **en ce que** les données de champ sont dépendantes de la position.

6. Procédé selon une des revendications précédentes, **caractérisé en ce qu'**un scénario agricole « inspection de cheptel » est une inspection d'un cheptel (3), préférentiellement **en ce que** des données individuelles d'un animal spécifique, en particulier un identifiant d'animal et/ou des données de gestation et/ou des données de composition alimentaire et/ou des données de production laitière et/ou des données de santé animale, sont affichées en tant qu'informations supplémentaires (Z).

7. Procédé selon une des revendications précédentes, **caractérisé en ce qu'**un scénario agricole « travail en champ » est un travail en champ avec une machine de travail agricole (4),
préférentiellement **en ce que** des données de champ, en particulier des données de produit et/ou des données de sol, en particulier une humidité de sol, et/ou des données de machine de la machine de travail agricole, en particulier une température et/ou un itinéraire planifié, sont affichées en tant qu'informations supplémentaires (Z), et/ou
**en ce que** l'appareil mobile (5) est relié à la machine de travail agricole (4) par l'intermédiaire d'une liaison radio (11), en particulier par WLAN ou Bluetooth, et l'agencement de commande (1) détermine sur la base de la liaison radio (11) que l'appareil mobile (5) se trouve dans le scénario agricole « travail en champ ».

8. Procédé selon une des revendications précédentes, **caractérisé en ce que** l'appareil mobile (5) est un smartphone ou une tablette ou un téléphone mobile ou un affichage tête haute d'une machine de travail agricole (4) ou des lunettes de données, et/ou **en ce que** la caméra (7) est intégrée dans l'appareil mobile (5) .

9. Procédé selon une des revendications précédentes, **caractérisé en ce que** l'utilisateur (B) effectue des entrées sur l'appareil mobile (5), **en ce que** les entrées sont associées par l'agencement de commande (1) à un objet représenté (O), les informations supplémentaires affichées (Z) sont modifiées par l'agencement de commande (1) sur la base des entrées et les informations supplémentaires modifiées (Z) sont stockées dans la banque de données (D), préférentiellement **en ce que** les données de champ sont modifiées en fonction de la position par des entrées de l'utilisateur (B) sur l'appareil mobile (5), et/ou **en ce que** les données individuelles de l'animal spécifique sont modifiées par des entrées de l'utilisateur (B) sur l'appareil mobile (5).

10. Procédé selon une des revendications précédentes, **caractérisé en ce que** l'utilisateur (B) dirige la caméra (7) sur un champ spécifique (2) et l'agencement de commande (1) détermine la présence d'un champ général (2) en tant qu'informations d'identification, et/ou **en ce que** l'agencement de commande (1) détermine, en particulier par l'intermédiaire d'un GPS, des informations de position de l'appareil mobile en tant qu'informations d'identification, préférentiellement **en ce que**, sur la base des informations d'identification, l'agencement de commande (1) détermine que l'appareil mobile (5) se trouve dans le scénario agricole « inspection de champ », plus préférentiellement **en ce que** l'agencement de commande (1) détermine, sur la base des informations d'identification, le champ spécifique (2) à partir d'une quantité de champs spécifiques prédéfinis (2) stockée
dans la banque de données (D), et l'agencement de commande (1) détermine à partir du champ spécifique (2) la quantité d'informations supplémentaires potentielles stockées dans la banque de données (D).

11. Procédé selon une des revendications précédentes, **caractérisé en ce que** l'utilisateur (B) dirige la caméra sur au moins un animal spécifique et l'agencement de commande (1) détermine la présence d'un animal général en tant qu'informations d'identification et/ou l'appareil mobile (5) est relié par l'intermédiaire d'une liaison radio (11), en particulier par RFID, avec un objet (13, 14) relié associé à l'animal, préférentiellement **en ce que** l'agencement de commande (1) détermine sur la base des informations d'identification et/ou de la liaison radio (11) que l'appareil mobile (5) se trouve dans le scénario agricole « inspection de cheptel », plus préférentiellement **en ce que** l'agencement de commande (1) détermine, en particulier sur la base de la liaison radio (11), l'animal spécifique à partir d'une quantité d'animaux spécifiques prédéfinis stockée dans la banque de données (D), et l'agencement de commande (1) détermine en fonction de l'animal spécifique la quantité d'informations supplémentaires potentielles (Z) stockées dans la banque de données (D).

12. Procédé selon une des revendications précédentes, **caractérisé en ce que** l'agencement de commande (1) détermine que l'appareil mobile (5) se trouve dans le scénario agricole « travail en champ » et l'agencement de commande (1) en déduit que l'appareil mobile (5) se trouve aussi dans le scénario agricole « inspection de champ ».

13. Procédé selon une des revendications précédentes, **caractérisé en ce que** les modules d'application (A) incluent au moins un module d'application (A) associé au scénario agricole « inspection de champ » et/ou un module d'application (A) associé au scénario agricole « inspection de cheptel » et/ou un module d'application (A) associé au scénario agricole « travail en champ », et/ou **en ce que** les modules d'application (A) sont fournis par divers fabricants, et/ou **en ce que** l'utilisateur (B) peut présélectionner divers modules d'application (A) et **en ce que** les scénarios agricoles prédéfinis sont associés aux modules d'application présélectionnés (A).

14. Appareil mobile (5) conçu pour une utilisation dans un procédé selon une des revendications 1 à 13, l'appareil mobile (5) comportant une caméra (7) et un affichage (8),
l'appareil mobile (5) étant conçu de façon qu'une application de serveur communique avec l'appareil mobile, l'application de serveur incluant un ou plusieurs serveurs sur lesquels est exécuté un programme informatique correspondant et sur lesquels est stockée une banque de données (D),
dans le programme de réalité augmentée, une reproduction de la réalité (R) générée par la caméra (7) et au moins une information supplémentaire (Z) stockée dans la banque de données (D) et associée à un scénario agricole étant affichées sur l'affichage (8) de l'appareil mobile (5) en étant superposées visuellement et
l'information supplémentaire respective (Z) étant associée au moins à un objet (O) du monde réel reproduit dans la reproduction de la réalité (R).

15. Application de serveur (6) comprenant une banque de données (D) conçue pour une utilisation dans un procédé selon une des revendications 1 à 13, l'application de serveur incluant un ou plusieurs serveurs sur lesquels est exécuté un programme informatique correspondant et sur lesquels est stockée la banque de données (D), l'application de serveur (6) communiquant avec un appareil mobile (5), l'application de serveur (6) incluant plusieurs modules d'application (A) auxquels un scénario agricole prédéfini est respectivement associé, et une quantité d'informations supplémentaires (Z) relative à chaque scénario agricole étant stockée dans la banque de données (D), l'agencement de commande (1) déterminant automatiquement dans lequel des scénarios agricoles prédéfinis se trouve l'appareil mobile (5).
